(19) **Europäisches Patentamt** / **European Patent Office** / **Office européen des brevets**

(11) **EP 3 264 322 A1**

(12) # EUROPEAN PATENT APPLICATION

(43) Date of publication:
**03.01.2018 Bulletin 2018/01**

(51) Int Cl.:
**G06K 9/00** (2006.01)

(21) Application number: **16177204.1**

(22) Date of filing: **30.06.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Deutsches Krebsforschungszentrum Stiftung des Öffentlichen Rechts**
**69120 Heidelberg (DE)**

(72) Inventors:
• **Maier-Hein, Lena**
  **69120 Heidelberg (DE)**
• **Kirchner, Thomas**
  **69115 Heidelberg (DE)**
• **Gröhl, Janek**
  **68169 Mannheim (DE)**

(74) Representative: **Lucke, Andreas**
  **Boehmert & Boehmert**
  **Anwaltspartnerschaft mbB**
  **Pettenkoferstrasse 22**
  **80336 München (DE)**

(54) **MACHINE LEARNING-BASED QUANTITATIVE PHOTOACOUSTIC TOMOGRAPHY (PAT)**

(57)     A method for estimating an optical property of a tissue (10) from a photoacoustic image (20) of the tissue (10) or parts thereof (12) using a machine learning algorithm, wherein the photoacoustic image (20) is obtained with a photoacoustic setup (30) and wherein the machine learning algorithm is configured to infer the optical property at least at one domain of the photoacoustic image (20) by means of a descriptor for said at least one domain, wherein at least part of the photoacoustic image (20) is partitioned in a plurality of domains (22) with respect to at least one parameter, wherein the at least one parameter corresponds to a physical property of the tissue (10), and wherein the variation of the at least one parameter within each domain is limited to a pre-determined value, such that each domain corresponds to a limited range of said physical property of the tissue (10), and a descriptor is determined for each of said at least one domain, wherein the descriptor for a given domain (V) comprises information related to the photoacoustic image (20) for each contributing domain $(v_{11},...,v_{33})$ of a set of contributing domains $(C_V)$, wherein the set of contributing domains $(C_V)$ comprises one or more domains other than said given domain (V).

Fig. 2

EP 3 264 322 A1

**Description**

FIELD OF THE INVENTION

The present invention is in the field of medical imaging. In particular, the present invention relates to photoacoustic imaging.

BACKGROUND OF THE INVENTION

Photoacoustic imaging is an emerging biomedical imaging modality that allows non-invasive structural, functional and molecular imaging. The prospect of obtaining images of soft tissues with fine spatial resolution, high sensitivity, and good specificity has increasingly directed the interest of the biomedical community to photoacoustic imaging. Recent studies have shown that photoacoustic imaging could be used in a number of promising biomedical applications, including brain lesion detection, haemodynamics monitoring, blood oxygenation mapping, functional brain imaging, skin melanoma detection, methemoglobin measuring, and tumour detection.

Photoacoustic imaging relies on the photoacoustic effect, that is, on the generation of sound waves through the absorption of light and its conversion to heat. Non-ionising, mostly short laser pulses are used as sampling light to irradiate a biological tissue. The local absorption of electromagnetic waves of sampling light in the tissue results in rapid heating, which subsequently leads to thermal expansion, whereby broadband acoustic waves are generated. Photoacoustic imaging is based on the detection of such acoustic waves and on their subsequent analysis in order to produce images of the biological tissue.

The energy of the sound waves produced at a region of the irradiated tissue as a result of the photoacoustic effect depends on the amount of sampling light absorbed therein, which in turn depends on the amount of sampling light reaching that region of the tissue and on the optical absorption thereof. Hence, knowledge about the distribution of sampling light in the tissue combined with knowledge about the energy of the sound waves produced therein by means of the photoacoustic effect can provide information about the optical absorption. Differences in the optical absorption between parts of the tissue are related to differences in the physiological properties or biochemical compositions thereof, whence details of the structure of the tissue can be inferred from the magnitude of the ultrasonic emission resulting from the photoacoustic effect. Thereby, the advantageous high contrast of optical imaging can be combined with the high-resolution achieved by ultrasonic imaging.

One of the current main goals in the development of photoacoustic imaging is to obtain multispectral tomographic images from measurements made using sampling light of multiple optical wavelengths. This allows obtaining information about the wavelength dependency of the measured or inferred quantities, which usually can be related to information about the molecular composition of the tissue. However, multispectral photoacoustic imaging that spans from ultraviolet to near infrared is technically involved in practice.

The images obtained by means of photoacoustic imaging are a representation of the energy density H absorbed by the tissue. This absorbed energy density H, related to the amount of sampling light absorbed, is a function of space and wavelength and is given by the product of the absorption coefficients $\mu$ accounting for the light absorption due to the chromophores in the tissue, the so-called light fluence $\Phi$, which is a measure of the radiance integrated over all directions and time, and of the Grüneisen coefficient $\Gamma$, which is related to thermodynamic properties of the tissue:

$$H = \Gamma \cdot \mu \cdot \Phi \qquad (1)$$

The fluence $\Phi$ and the absorption coefficients $\mu$ are intertwined in a nontrivial way due to the dependence of the first on the chromophore concentrations, and hence on the absorption coefficients themselves. Further, the absorption coefficient $\mu$, and thereby also the fluence, also depend on the wavelength of the sampling light and on the position x in the tissue, due to the effects of light scattering inside the tissue, described by a scattering coefficient $\mu_s$. When taking all functional dependencies into account, equation 1 takes the form:

$$H(x, \lambda) = \Gamma(x,T) \cdot \mu(x,\lambda) \cdot \Phi(x,\lambda,\mu, \mu_s) \qquad (2)$$

where T is the temperature and $\mu_s$ a scattering coefficient. Given the interdependency of the fluence $\Phi$ and the absorption coefficient $\mu$, this equation may not be solved by conventional linear methods or matrix inversion, even when assuming, as is usually done for constant temperatures T, approximately constant values of the Grüneisen coefficient $\Gamma$ and the scattering coefficient $\mu_s$ throughout the tissue. As a result of this, the correct estimation of the fluence $\Phi$ poses a major

obstacle when it comes to the determination of properties of the tissue under observation by means of photoacoustic imaging. In particular, the wavelength dependence of the fluence, which is an important prerequisite for numerous biomedical applications, takes a yet-to-be-solved technical problem to an ulterior degree of difficulty.

Traditionally, photoacoustic measurements have been related to physiological properties of the tissue under observation by means of analytical or numerical models of light propagation through the tissue to approximate the fluence. The state-of-the-art solutions to obtain multispectral photoacoustic images display limited reliability and typically rely on highly complex hardware setups and on time-consuming computational methods that make multispectral photoacoustic imaging only suitable for retrospective off-line image analysis. Existing methods to estimate optical properties of a tissue from a photoacoustic image thereof are hence doomed to be either based on simplistic models which do not provide an acceptable level of accuracy or based on cumbersome models that result in very slow working paces.

In view of the above, there is room for technical improvements in the field of photoacoustic imaging regarding the efficiency and reliability of the methods employed for approximating the fluence, in particular in view of an application to multispectral photoacoustic imaging.

## SUMMARY OF THE INVENTION

The problem underlying the invention is to provide a method, a computer program product and an apparatus for estimating an optical property of a tissue from a photoacoustic image in a fast, simple and accurate manner allowing real-time, in vivo application. This problem is solved by a method according to claim number 1, a data-processing apparatus according to claim 12, computer program products according to claims 13 and 14, and by a computer-readable storage medium according to claim 15. Preferred embodiments of the invention are defined in the dependent claims.

One aspect of the invention is related to a method for estimating an optical property of a tissue from a photoacoustic image of the tissue using a machine learning algorithm, wherein the photoacoustic image is obtained with a photoacoustic setup and wherein the machine learning algorithm is configured to infer the optical property at least at one domain of the photoacoustic image by means of a descriptor for each of said at least one domains.

Herein, a "domain" is understood as a partition of the photoacoustic image according to a parameter corresponding to a physical property of the tissue. A "machine learning algorithm" is understood to be any algorithm able of autonomously developing and improving the ability to carry out a given task based on learning input. In the present case, the task in question is that of inferring a target quantity - the optical property in this case - from input data causally connected to the target quantity - herein the photoacoustic image - without knowledge of the precise causal connection, that is, of the physical law or mathematical functional dependency, governing the interdependence of the input data and the target quantity.

Learning input refers herein to information, be it measured, analytically or numerically computed, and/or simulated, in which both input data and values of the target quantity causally related to the input data are encoded. The machine learning algorithm analyses the learning input and extracts from it information about the underlying causal connection between the input data and the target quantity, such that a contribution is made towards the development and improvement of the ability to carry out the task in question. In the present case, learning input typically comprises photoacoustic images of a tissue and values of the optical property thereof.

A "photoacoustic image" is understood herein as a collection of data, be it raw or processed, obtained from the ultrasonic waves produced at the tissue as a result of the photoacoustic effect when irradiating the tissue with sampling light. A "photoacoustic set up" in the sense of the present invention is any apparatus or device configured for obtaining photoacoustic images. A "descriptor" is a vector of features containing information relative to a given object, which is used by the machine learning algorithm to characterise said given object. In the case of the present invention, the machine learning algorithm uses a descriptor to characterise a given domain of the photoacoustic image.

Unlike in the prior art, according to which in photoacoustic measurements the fluence has been determined employing deterministic analytical or numerical models of light propagation, or, in other words, by a "physicists approach", in the present invention the optical property of a tissue is estimated from the photoacoustic image by means of a machine learning algorithm.

Machine learning is a technique in which a computer progressively develops and improves the ability to carry out a task by analysing a sufficient amount of learning input. The present invention presents a novel way of solving the problem elucidated above with regard to equation (2), namely of estimating the value of an optical property of a tissue from a photoacoustic image thereof. The solution according to the invention is based on letting a machine learning algorithm develop the ability to do so in an efficient way by optimising the way in which the learning input and the input data are processed by the machine learning algorithm. The inventors have confirmed that surprisingly, by using a machine learning algorithm, the desired optical properties, such as the absorption coefficient or the fluence at a given region of the tissue, can be determined with great accuracy based on a reasonable amount of learning input.

A crucial step in the machine learning processing of input data and of learning input is the choice of appropriate descriptors to represent the objects relevant to the processing. A descriptor usually comprises a vector of features, which are used to characterise the object they are associated to. Developing a descriptor suitable for analysing data with a given method of analysis requires the selection of a set of features or attributes that is adequate for the selected method. This selection is critical to the success of a descriptor. The use of too many attributes or of unsuitable attributes may lead to overfitting or to spurious associations that may result in false conclusions. The present invention presents a class of descriptors to be used for the specific problem at hand which allows for the successful application of machine learning to the purpose of photoacoustic imaging.

In the method of the invention, at least part of the photoacoustic image is partitioned in a plurality of domains with respect to at least one parameter, wherein the at least one parameter corresponds to a physical property of the tissue, and wherein the variation of the at least one parameter within each domain is limited to a predetermined value, such that each domain corresponds to a limited range of said physical property of the tissue. Hence, when the photoacoustic image is partitioned in domains with respect to a given parameter corresponding to a given physical property of the tissue, a map between the space of values of the given parameter and the space of values of the physical property of the tissue is established. Thereby, each partition or domain of the photoacoustic image corresponds to a limited range of the physical property of the tissue. If, for example, the photoacoustic image is partitioned with respect to a parameter X, which corresponds to a physical property P of the tissue, there is a one-to-one map between the values of X and values of P. The photoacoustic image is then partitioned in domains, namely in limited subsets of the set of possible values of X, which each correspond to a limited range of the physical property P, that is to a given subset of the set of possible values of P.

According to the method of the invention, the descriptor for a given domain comprises information related to the photoacoustic image for each contributing domain of a set of contributing domains, wherein the set of contributing domains comprises one or more domains other than said given domain. Hence, each given domain of the photoacoustic image is related to a set of surrounding domains of the photoacoustic image, which make up the set of contributing domains of that given domain. These may comprise neighbouring domains of the given domain of the photoacoustic image but may also comprise more distant domains. The descriptor of a given domain hence comprises information related to the photoacoustic image corresponding to a number of domains other than said given domain. Said given domain may also be considered as part of the set of contributing domains. This way, the machine learning algorithm may characterise a given domain by means of a descriptor that accounts for the interdependency between different domains due to the non-local effects involved in light propagation through different parts of the tissue.

The use of such a descriptor to process photoacoustic images of a tissue has been shown by the inventors to enable the machine learning algorithm to develop and improve the ability of inferring an optical property of the tissue from a photoacoustic image thereof. This allows for a fast estimation of the optical property using the machine learning algorithm, notably outperforming the state-of-the-art time-consuming photoacoustic image processing methods for multispectral photoacoustic imaging. The machine learning algorithm of the present invention constitutes a pioneering solution which sets a new benchmark in terms of accuracy and reliability regarding the determination of an optical property, for instance the fluence, on the purposes of photoacoustic imaging. In addition, thanks to the much higher speed of estimation, the method of the invention further opens the door to real-time in vivo application, which offers a great advantage over the existing methods, which are only suitable for retrospective offline image analysis.

Further, the machine learning algorithm may delineate irrelevant data, so as to mitigate the hardware processing requirements and to accelerate the entire processing. Along the same lines, the use of a machine learning algorithm constitutes a software-based solution which allows adapting existing photoacoustic setups to implement the method of the invention. This way, highly complex hardware setups are made unnecessary, while a cost-effective and simple way of upgrading available setups by adapting them to implement the method of the invention is provided.

In preferred embodiments of the invention, the descriptor for a given domain further comprises, for each contributing domain of the set of contributing domains, information related to the location of said given domain and said contributing domain with regard to the photoacoustic setup. This information may allow accounting for differences in the photoacoustic measurements corresponding to different domains of the photoacoustic image due to the different locations of said different domains with respect to the photoacoustic setup. Further, different balances in the interdependencies between a domain and the contributing domains thereof for domains corresponding to different parts of the tissue may also be accounted for by such a descriptor. By referring the information related to the location of said given domain and said contributing domain to the photoacoustic setup, it is possible to identify the individual domains with respect to the photoacoustic setup, namely irrespectively of the properties, spatial disposition or component constitution of the tissue.

According to preferred embodiments of the invention, the information related to the location of said given domain and each of said contributing domains with regard to the photoacoustic setup is in the form of a contribution specifier, wherein the contribution specifier for a contributing domain defines a relationship between said contributing domain and said given domain, wherein the relationship reflects characteristics of the photoacoustic setup. Preferably such characteristics comprise the geometry of the photoacoustic setup, the properties of the sampling light, or the effects of light propagation through the tissue. For instance, the geometry of the photoacoustic setup determines the way in which the sampling light reaches different parts of the tissue, which affects the magnitude of the energy absorbed therein, as elucidated above. This is reflected in differences in the degree of correlation between different domains for a particular setup geometry, which may be encoded in the corresponding contribution specifiers.

Such a descriptor provides an efficient way of handling data for the machine learning algorithm to correctly estimate the optical property of the tissue taking into account the aforementioned non-local effects. By means of the contribution specifiers, fixed conditions related to the geometry of the photoacoustic setup, the properties of the sampling light, and the effects of light propagation through the tissue may be accounted for by the descriptor through the contribution specifiers. This allows a separated processing of the part of the descriptor in which said fixed conditions are reflected, such that, for example, in the case that several photoacoustic images are produced under identical or similar such fixed conditions, previously determined contribution specifiers may be used again so that they need not be determined anew for each photoacoustic image. Other factors influencing light propagation in the tissue and hence the interdependency of the photoacoustic image at different domains, like the specific geometry of the photoacoustic setup, the spatial distribution of different organic components with different densities in the tissue, or the presence of localised concentrations of a particular organic component, like for instance tumours, may be accounted for by the contribution specifiers as well.

According to preferred embodiments of the invention, the information related to the photoacoustic image comprises a value of a photoacoustic signal, wherein the photoacoustic signal is evaluated at least at some of said plurality of domains, and wherein the value of the photoacoustic signal at a given domain is a measure of the energy of the sound waves resulting from the photoacoustic effect in that given domain. Hence the value of the photoacoustic signal at one domain is related to the amount of sampling light used for obtaining the photoacoustic image absorbed in the part of the tissue represented by said given domain. In other words, the photoacoustic signal is a quantity proportional to the energy density H of equations (1) and (2). The photoacoustic signal may hence provide information about the amount of sampling light absorbed in the corresponding domain of the photoacoustic image, which combined with the information about the characteristics of the photoacoustic setup enclosed in the contribution specifiers, may provide a way of non-locally correlating the domains and hence of correctly estimating the optical property taking into account, for instance, the effects of light propagation through different parts of the tissue.

Thus, the descriptor for a domain of the photoacoustic image whose set of contributing domains comprises, say, N contributing domains is formed by a vector object comprising N tuples, wherein each tuple contains, for each contributing domain, a contribution specifier and information related to the photoacoustic image at said contributing domain.

For instance, if the photoacoustic image is partitioned in M domains, the machine learning algorithm is configured to infer the optical property at least at one of the M domains, namely at K domains, wherein $K \leq M$. Then, the photoacoustic image is characterised by K descriptors, each comprising N tuples. A total number of different values smaller than N x K might be used by exploiting the symmetry of the photoacoustic image or of the photoacoustic setup.

In a preferred embodiment of the invention, the method further comprises a training process of the machine learning algorithm, wherein during the training process the machine learning algorithm analyses a sequence of training images and learns how to infer the optical property of the tissue from a photoacoustic image of the tissue using said descriptors, wherein the sequence of training images comprises photoacoustic images of a tissue and values of the optical property of said tissue. The sequence of training images constitutes the learning input from which machine learning algorithm extracts information about the underlying causal connection between the photoacoustic images and the known values of the optical property.

Note, however, that while a certain amount of training is of course necessary for the machine learning algorithm to obtain reliable results, the training as such needs not be carried out by the user of the method of the invention. Instead, the method in the general sense can be also carry out based on an "already trained machine learning algorithm", where the training step as such is not part of the protected method. More shall be said on this below.

By means of the training process, the machine learning algorithm of the invention develops and improves the ability to estimate the value of the optical property from a photoacoustic image in a way characteristic of this kind of algorithm. This allows the algorithm to train on the sequence of training images, be it by itself or aided by a human user, which makes a full model understanding of the biological system of the tissue unnecessary for the purposes of photoacoustic imaging.

The machine leaning algorithm may further pinpoint unknown causal relationships in the input data or in the learning input, for instance by noting previously interdependencies between features of the tissue. Then the machine learning algorithm may autonomously evolve its design so as to accommodate the new findings. This has as a potential huge impact on the purposes of predictive analysis and diagnosis. For example, the machine learning algorithm may learn to detect the presence of anomalies, damages or tumours in the tissue from unknowingly related features of the corresponding photoacoustic images.

The sequence of training images may comprise simulated photoacoustic images obtained at least in part by computer simulation, and/or real photoacoustic images obtained by means of photoacoustic imaging. Preferably, simulated training images may be generated with Monte Carlo methods. For example, training images may be obtained by departing from a simulated tissue with known values of the optical property and simulating the process of photoacoustic imaging so as to obtain a corresponding simulated photoacoustic image. The sequence of training images may comprise images of the same tissue or of different issues. The inclusion of simulated photoacoustic images in the sequence of training images allows generating a large amount of learning input for the machine learning algorithm to learn from in a very short time compared to the time that would be required to generate a comparable amount of learning input by real photoacoustic measurements. The inclusion of real photoacoustic images of a real tissue with known values of the optical property allows incorporating into the sequence of training images real, possibly unexpected, features of the tissue, which the machine learning algorithm can learn to recognise.

The method of the invention may further comprise transfer learning means to deal more efficiently with training images. For example, transfer learning means may be employed such that the inclusion of real photoacoustic images in the sequence of training images can be used to identify practically occurring features and/or descriptors so as to evaluate simulated data in accordance. This way, proportions or variabilities in the data of the training images not corresponding to realistic situations may be compensated. In particular, simulated descriptors found to correspond to practically occurring descriptors may be dominantly weighted over less realistic ones. Thereby, the training is based on learning input that better resembles really occurring photoacoustic images of real tissues.

Further, when estimating the optical property at a given domain, the algorithm may predominantly weight data from domains of previous training images that resemble the given domain according to selected properties. This may include, for instance, taking into account the similarity of the respective domains with respect to their composition or structure. Further, it is possible to predominantly weight those pieces of training data, i.e. selected training images or domains thereof, that have a potentially higher relevance for a given kind of tissue or photoacoustic image, taking into account the properties of the tissue under observation and/or the specifications of the photoacoustic setup.For example, when determining the descriptor for a domain corresponding to a region of the tissue with a high accumulation of fat, it may be beneficial to learn from previous training images by focusing on those domains of said training images which also display a high accumulation of fat. As a result, the accuracy of the method for estimating the optical property can be increased.

According to preferred embodiments of the invention, the method further comprises a measuring process after the training process, wherein during the measuring process, the machine learning algorithm infers the optical property at least at one domain of the photoacoustic image from the descriptor determined for said at least one domain. Thereby, the experience accumulated by the machine learning algorithm during the training process can be exploited and used in the estimation of the optical property of a new photoacoustic image. In particular, the measuring process may be carried out in vivo.

Preferably, the measuring process may comprise determining for the descriptor for the at least one domain only the corresponding information related to the photoacoustic image for each contributing domain of a set of contributing domains, or the corresponding photoacoustic signal. Instead, the information related to the location of said given domain and each of said contributing domains with regard to the photoacoustic setup for each contributing domain of the set of contributing domains or the contribution specifier for each contributing domain of the set of contributing domains may preferably be determined before the measuring process. For example, the contribution specifiers may be determined during a training process preceding the measuring process, The contribution specifiers may however also or alternatively be determined before the measuring process by means of an analytic model of light-tissue interaction.

This way, the machine learning algorithm may learn during the training process how to take into account fixed conditions, like the geometry of the photoacoustic setup, and hence to determine the information related to the location of said given domain and each of said contributing domains with regard to the photoacoustic setup for each contributing domain of the set of contributing domains or the contribution specifier for each contributing domain of the set of contributing domains. Subsequently, only the information related to the photoacoustic image for each contributing domain of a set of contributing domains, or the corresponding photoacoustic signal has to be obtained in order to obtain the estimation of the optical property of the tissue in question. This provides the benefit of speeding up the method of the invention, since the first part of the descriptor of the at least one domain needs not be determined in real time.

According to preferred embodiments of the invention, the descriptor for a given domain is determined as a histogram registering values of the contribution specifiers and of the photoacoustic signals. For example, the machine learning algorithm may be, as described above, configured to infer the optical property at K domains and each set of contributing domains may be made up of N contributing domains. A given domain at a given training image may then be characterised by a 2N-tuple of values comprising N pairs of values, that is, one pair of values for each of the N contributing domains of the corresponding set of contributing domains, wherein each pair of values comprises a value of the corresponding contribution specifier and a value of the corresponding photoacoustic signal. Such a 2N-tuple can then be processed and transformed into a histogram by discretising the feature space of contribution specifier and photoacoustic signal in a number of bins. The bins may be of different sizes, for example when partitioned according to a logarithmic scale. The number of bins is the same for all domains and for all training images. After discretising, the number of pairs of values in each bin is counted and stored of in the histogram. The descriptor for a given domain is then determined as a histogram. The inventors have shown that the use of such histograms allow the machine learning algorithm to process photoacoustic images of a tissue to develop and improve the ability of inferring an optical property of the tissue in a manner that notably outperforms previously known methods in terms of speed and accuracy.

When using such histograms, it is not required to keep track of the particular values of the contribution specifiers and values of the photoacoustic signal for each of the contributing domains of the domain in question. Instead, a given domain may be characterised by means of the histogram by the population distribution of said values, which may allow reducing the amount of data to be stored as a descriptor by several orders of magnitude. This allows characterising the domains by a much smaller number of values to be stored, thereby reducing the susceptibility of the obtained estimation of the optical property to noise, saving computational resources and significantly reducing processing time compared to previously known methods. This way, the method of the invention allows analysing a larger amount of training data, for instead of training on N entire training images as traditionally done in the prior art, the algorithm can be trained on N x M training examples. Alternatively, the use of such histograms may allow, for given computational resources, characterising the domains by descriptors taking into account a larger amount of contributing domains per domain and/or partitioning the photoacoustic image in a larger number of domains.

In a preferred embodiment of the invention, the machine learning algorithm analyses, for one or more training images of the sequence of training images, a vector object comprising histograms for each of said at least one domain and a vector object comprising corresponding values of the optical property at each of said at least one domain. By means of such vector objects, the machine learning algorithm may learn to process at once information comprising all descriptors for all domains from all training images of a sequence of training images and information comprising values of the optical property for all domains at all training images of a sequence of training images. For instance, a vector object comprising histograms for each of said at least one domain and for each training image of said sequence of training images and a vector object comprising corresponding values of the optical property at each of said at least one domain and for each training image of said sequence of training images may be fed to the machine learning algorithm during or after the training process, such that the machine learning algorithm learns how to infer an optical property of a tissue from a photoacoustic image of the tissue from such vector objects. In the course of processing such vector objects, the machine learning algorithm may split the data in different ways, for instance according to a bootstrap aggregating technique. By means of such a histogram, the extent to which the machine learning algorithm may profit from previous learning experience is maximised. For example, redundant information may be obtained for a given domain, which may be employed to improve the accuracy and the confidence of the estimation of the optical property therein. As a result, the estimation of the optical property according to the invention can be rendered more reliable.

According to preferred embodiments of the invention, the contribution specifier for a given contributing domain of a given domain may be related to the degree to which the value of the photoacoustic signal and/or of the optical property at said given contributing domain is related to the value of the photoacoustic signal and/or of the optical property at said given domain for a given photoacoustic setup. Preferably, the contribution specifier might be related to the likelihood that sampling light used for obtaining the photoacoustic image with a given photoacoustic setup reaching said given domain has previously reached said contributing domain. Further, the contribution specifier may comprise information related to the confidence of the inferred optical property at said given domain.

This way, the contribution specifier may account for the non-local effects involved in light propagation through different parts of the tissue when a given photoacoustic setup is used. For example, it may be taken into account, that a domain of the photoacoustic image corresponding to a region of the tissue situated closer to a source of light of the photoacoustic setup is very likely to absorb more sampling light than a domain of the photoacoustic image corresponding to another region of the tissue situated farther away from said source of light. Other factors influencing light propagation, like the different molecular composition of the tissue, may be accounted for by the contribution specifiers as well.

In a preferred embodiment of the invention, the machine learning algorithm may further be configured for estimating a confidence of an optical property at each of the at least one domains of the photoacoustic image. This way, the confidence of the estimation of the optical property at a given domain can be taken into account when relating said domain to another domain. For example, it is possible to make use of spatial regularisation so as to remove or at least undervalue outlying data. Further, it is possible to employ the estimated confidence so that contributing domains having a greater confidence may be dominantly taken into account when determining the descriptor and/or the value of the optical property of a domain. For example, values of the confidence may be combined with the values of the estimated optical property to generate a 3D volume estimation of the optical property from sequences of 2D measurements.

In addition, the confidence may be propagated to subsequent calculations. For instance, when inferring an oxygenation estimation in the tissue from the estimated optical property, the confidence of the estimation of the optical property at a given domain can be propagated to obtain the confidence of the computed oxygenation estimation at that given domain. A further possibility consists in using the estimated confidence to weight a parameter of choice depending on the value of a property of a domain or group of domains according to the confidence thereof. The confidence value may further be included in the descriptors.

In preferred embodiments of the invention, at least one contribution specifier is computed by means of an analytic model or a simulation for a given photoacoustic setup. For example, in the case that the geometry of the photoacoustic setup allows a simplified point-light-source model to be employed, this may be exploited to determine the contribution specifiers so as to speed up the process of estimating the optical property. However, it is also possible that at least one contribution specifier is computed by means of computer simulation. Preferably, a Monte Carlo method may be used for computing said at least one contribution specifier.

According to preferred embodiments of the invention, the at least one parameter comprises a parameter corresponding to any of a spatial dimension, a frequency, and a temperature. Preferably, the photoacoustic image is partitioned with respect to three parameters, wherein said three parameters correspond to three spatial dimensions of the tissue, were in particular, the domains correspond to voxels.

Hence each of the domains of the photoacoustic image may correspond to a spatial region of the tissue. However, other parameters may be used for partitioning the photoacoustic image, in which case a domain of the photoacoustic image corresponds to a region of the tissue falling within the corresponding range with respect to said parameters. In particular, it is possible to partition the photoacoustic image in frequency space, so as to possibly take use of Fourier transform methods for estimating the optical property. A combination of spatial, frequency and other parameters is possible as well.

In preferred embodiments of the invention, the optical property is estimated from a sequence of photoacoustic images of the tissue obtained using sampling light of different wavelengths. By probing the tissue at different wavelengths, values of the optical property corresponding to different wavelengths of the sampling light can be estimated, which is an important prerequisite for the estimation of optical properties such as oxygenation. Multispectral imaging may, for example, allow revealing subsurface structures, which are not visible otherwise. Preferably, the sampling light used for obtaining the photoacoustic image comprises light of wavelengths between 400 nm and 1600 nm, although sampling light in other parts of the electromagnetic spectrum, as well as particle beams, may be used as well.

Further, the machine learning algorithm may infer properties of the tissue directly from a set of photoacoustic images obtained using sapling light of different wavelengths. For example, oxygenation could be estimated without the need to resort to an intermediate estimation of an optical property for each of the wavelengths used to obtain the photoacoustic images of said set of photoacoustic images.

According to preferred embodiments of the invention, the optical property corresponds to or is at least related to the absorption coefficient and/or the optical absorption. The optical property may also correspond or at least be related to the fluence. Once the absorption coefficient or the fluence of the tissue have been estimated, other properties of the tissue may be inferred therefrom, like for example, chromophore concentrations, oxygenation, or fat (i.e. lipid) concentration, on which functional and molecular imaging can be based.

In a preferred embodiment of the invention, the photoacoustic image is obtained by means of any of photoacoustic tomography, photoacoustic microscopy, or photoacoustic elastograhpy

According to preferred embodiments of the invention, the machine learning algorithm comprises any of a deep learning algorithm, a random forest algorithm, a support vector machine algorithm, and a convolutional neural network algorithm.

A further aspect of the invention relates to a data-processing apparatus configured for carrying out any of the methods disclosed above.

A further aspect of the invention relates to a computer program product including executable code which when executed on a computer carries out any of the methods disclosed above.

A further aspect of the invention relates to a computer program product including executable code which when executed on a computer estimates an optical property of a tissue from a photoacoustic image of the tissue or a part thereof using a machine learning algorithm, wherein the photoacoustic image is obtained with a photoacoustic setup and wherein the machine learning algorithm is configured to infer the optical property at least one domain of the photoacoustic image by means of a descriptor for each of said at least one domains. Therein, at least part of the photoacoustic image is partitioned in a plurality of domains with respect to at least one parameter, wherein the at least one parameter corresponds to a physical property of the tissue, and wherein the variation of the at least one parameter within each domain is limited to a pre-determined value, such that each domain corresponds to a limited range of said physical property of the tissue. Further, the descriptor for a given domain comprises information related to the photoacoustic image for each contributing domain of a set of contributing domains, which information is obtained from the photoacoustic image, and a contribution specifier for each contributing domain of said set of contributing domains, which contribution specifier is comprised in the executable code, wherein the set of contributing domains comprises one or more domains other than said given domain, and wherein the contribution specifier for a contributing domain defines a relationship between said contributing domain and said given domain, wherein the relationship reflects characteristics of the photoacoustic setup.

Preferably such characteristics comprise the geometry of the photoacoustic setup, the properties of the sampling light, or the effects of light propagation through the tissue. For instance, the geometry of the photoacoustic setup determines the way in which the sampling light reaches different parts of the tissue, which affects the magnitude of the energy absorbed therein, as elucidated above. This is reflected in differences in the degree of correlation between different domains for a particular setup geometry, which may be encoded in the corresponding contribution specifiers.

Hence such computer program product contains an executable code which has already been trained and which can use the accumulated experience to infer the optical property from a new photoacoustic image according to one of the methods described above.

A further aspect of the invention relates to a computer-readable storage medium comprising any of the computer program products disclosed above.

BRIEF DESCRIPTION OF THE DRAWINGS

Fig. 1    shows a schematic representation of the different elements to which the method of the invention refers:

a. shows a tissue and a part thereof, from which a photoacoustic image is obtained;
b. shows the photoacoustic image of the tissue obtained and the domains it is partitioned in; c. shows a representation of a vector object and of a descriptor according to the invention.

Fig. 2    shows a schematic representation of the geometry of a photoacoustic setup used to obtain a photoacoustic image of the tissue of Fig. 1.

Fig. 3    illustrates the determination of a descriptor as a histogram.

Fig. 4    shows a representation of a photoacoustic image analysed by the machine learning algorithm during a training process.

DESCRIPTION OF THE PREFERRED EMBODIMENTS

For the purposes of promoting an understanding of the principles of the invention, reference will now be made to a preferred embodiment illustrated in the drawings, and specific language will be used to describe the same. It will nevertheless be understood that no limitation of the scope of the invention is thereby intended, such alterations and further modifications in the illustrated apparatus and such further applications of the principles of the invention as illustrated therein being contemplated as would normally occur now or in the future to one skilled in the art to which the invention relates.

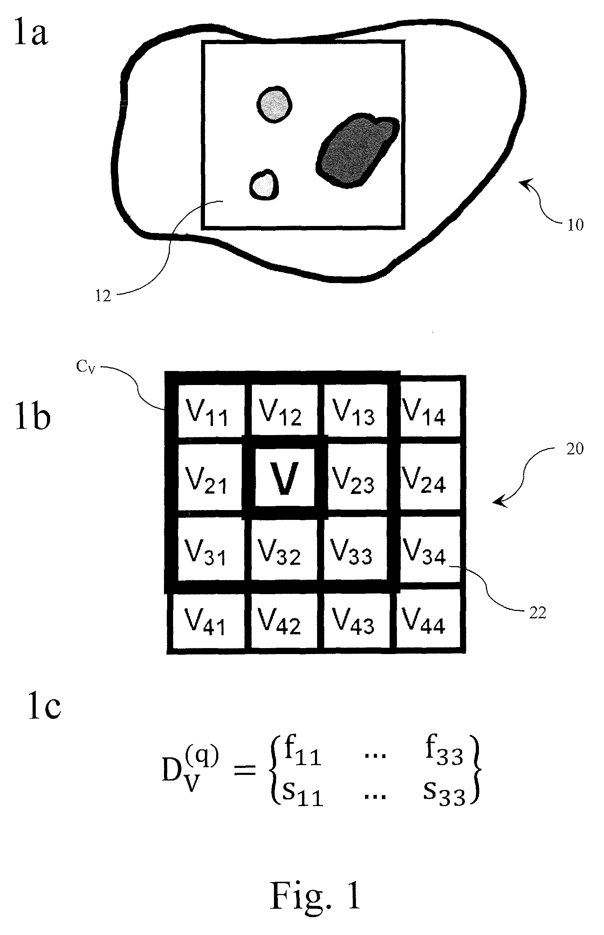

Figure 1 shows a schematic representation of a tissue 10 from which a photoacoustic image 20 can be obtained. The tissue 10 is treated with a photoacoustic setup so as to obtain a photoacoustic image 20 of a part of the tissue 12. According to the invention, a particular kind of descriptor is used by a machine learning algorithm to process the photoacoustic image 20 so was to infer from it an optical property of the tissue 10. In the shown embodiment, the photoacoustic image 20 can be a representation of raw or processed data obtained from the sound waves produced in the part of the tissue 12 covered the photoacoustic image 20 in the course of photoacoustic imaging.

For the purposes of explanation, it shall be initially assumed that the photoacoustic image 20 corresponds to any photoacoustic image processed by the machine learning algorithm of the invention. In particular, the photoacoustic image 20 can correspond to a training image, real or simulated, which is processed by the machine learning algorithm of the invention in the course of a training process with the scope of developing and improving the ability to estimate the value of an optical property of a tissue from a photoacoustic image thereof. However, the photoacoustic image 20 can also correspond to a photoacoustic image processed by the machine learning algorithm of the invention in the course of a measuring process whereby the machine learning algorithm infers an optical property of a tissue from a photoacoustic image thereof. As will become clear in the course of explanation, the description to follow may apply to any photoacoustic image processed by the machine learning algorithm.

The photoacoustic image 20 shown in Fig. 1b is partitioned in a plurality of domains 22 with respect to three parameters, wherein said three parameters correspond to the three spatial dimensions of the tissue 10. Hence each of the domains 22 of the photoacoustic image 20 corresponds to a voxel, that is, to a data representation of a limited spatial region of the tissue 10. However, the photoacoustic image 20 could also be partitioned with respect to other parameters, like for example mode frequencies in the context of a Fourier analysis processing, in which case each of the domains 22 of the photoacoustic image 20 would correspond to a region in frequency space associated to a corresponding spatial region of the tissue 10 in the dual space. Note that the representation in the figure is two-dimensional on illustration proposes only but a three-dimensional photoacoustic image 20 of the three-dimensional tissue 10 is hereby meant.

Each of the domains 22 is assigned a vector object $D_V^{(q)}$, shown in Fig. 1c, by means of which the machine learning algorithm characterises each of the domains 22. For a given domain, in this case, for each voxel, V, a set of contributing domains $C_V$ is determined that comprises one or more domains $V_{11},...,V_{33}$ other than said given domain V and the contributing domain $V_{22}$, which corresponds to said given domain V itself. Hence, in the embodiment shown, the set of contributing domains of a given voxel V comprises 9 contributing domains $v_{11},...,v_{33}$.

In the embodiment shown, the information related to the photoacoustic image is comprised in a value of a photoacoustic signal $s_{ij}$, wherein the photoacoustic signal $s_{ij}$ is evaluated at each of the domains 22, and wherein the value of the photoacoustic signal $s_{ij}$ at a given domain is a measure of the energy of the sound waves resulting from the photoacoustic effect in the region of the tissue 10 corresponding to that given domain. Further, in the embodiment shown, the vector object $D_V^{(q)}$ for each of the domains 22 comprises, for each contributing domain of the corresponding set of contributing domains, information related to the location of said given domain and each of said contributing domains with regard to the photoacoustic setup in the form of a contribution specifier $f_{ij}$, wherein the contribution specifier $f_{ij}$ for a contributing domain $v_{ij}$ defines a relationship between said contributing domain $v_{ij}$ and the corresponding given domain V, wherein the relationship reflects characteristics of the photoacoustic setup, like the geometry thereof, the properties of the sampling light, and/or the effects of light propagation through the tissue.

Since the vector object $D_V^{(q)}$ comprises information related to the location of said given domain and each of said contributing domains with regard to the photoacoustic setup in the form of a contribution specifier $f_{ij}$, the domains 22 can be uniquely identified with respect to the photoacoustic setup, that is, irrespectively of the properties, spatial disposition or component constitution of the tissue 10.

Hence the vector object $D_V^{(q)}$ for the given domain V shown in Fig. 1c comprises 9 2-tuples, wherein each 2-tuple comprises information related to the photoacoustic image 20 for a contributing domain $v_{ij}$ of the set of contributing domains $C_V$ in the form of a value of a photoacoustic signal $s_{ij}$ and a contribution specifier $f_{ij}$ for that same contributing domain $v_{ij}$, wherein the contribution specifier $f_{ij}$ defines a relationship between the contributing domain $v_{ij}$ and the domain V, wherein the relationship reflects characteristics of the photoacoustic setup

These characteristics comprise the geometry of the photoacoustic setup, the properties of the sampling light and the effects of light propagation through the tissue. The contribution specifier $f_{ij}$ for each of the contributing domains $v_{ij}$ of the domain V is related to the degree to which the value of the photoacoustic signal $s_{ij}$ is related to the value of the photoacoustic signal at the domain V for a given photoacoustic setup. In particular, the contribution specifier $f_{ij}$ for a given domain V and for a given contributing domain $v_{ij}$ is related to the likelihood that light used for obtaining the photoacoustic image 20 with a given photoacoustic setup reaching said given domain V has previously reached the contributing domain $v_{ij}$. For instance, the contributing domain $v_{11}$ is taken into account when estimating the optical property at the domain V by letting the value of the photoacoustic signal $s_{11}$ measured at the domain $v_{11}$ be included in the vector object $D_V^{(q)}$ according to the contribution specifier $f_{11}$, wherein the contribution specifier $f_{11}$ is related to the degree to which the value of the photoacoustic signal $s_{11}$ at the contributing domain $v_{11}$ is related to the value of the photoacoustic signal at the domain V for the employed photoacoustic setup, taking into consideration the geometry of the photoacoustic setup, the properties of the sampling light and the effects of light propagation through the tissue.

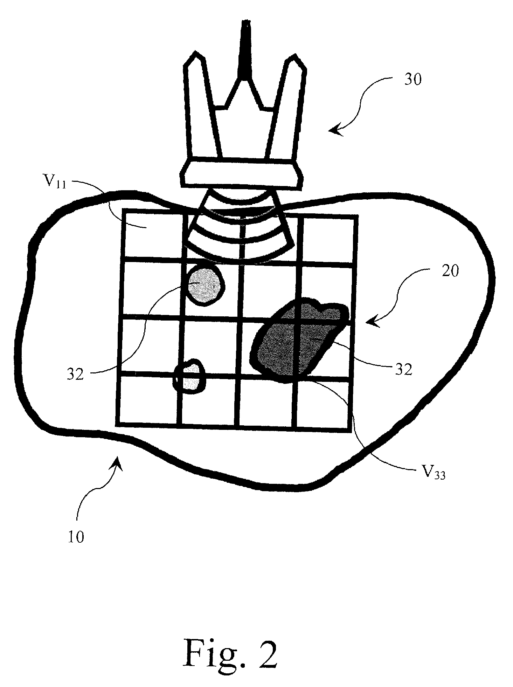

Figure 2 shows a schematic representation of the geometry employed for obtaining the photoacoustic image 20 of the tissue 10 using a photoacoustic setup 30. In the figure, the photoacoustic image 20 is superimposed on the tissue 10 on illustrative purposes but corresponds to the part of the tissue 12 it covers as seen in Fig. 1. As shown in Fig. 2, the photoacoustic setup 30 sends sampling light to the tissue 10 for obtaining the photoacoustic image 20 from an upper side with respect to the position of the tissue 10. As a result of this particular geometry, more sampling light is likely to be absorbed in the region of the tissue 10 corresponding e.g. to the domain $v_{11}$ of the photoacoustic image 20 than in the region of the tissue 10 corresponding to the domain $v_{33}$. Therefore, the value of the photoacoustic signal $s_{11}$ at the domain $v_{11}$ can be greater than the value of the photoacoustic signal $s_{33}$ at the domain $v_{33}$ even if the value of the optical property of interest, for example of the optical absorption is higher at $v_{33}$ than at $v_{11}$. The fact that this be due to the fluence in $v_{11}$ being greater than the fluence in $v_{33}$ is captured by the corresponding values of the contribution specifiers $f_{11}$ and $f_{33}$. Such information can then be used by the machine learning algorithm during the training process to learn how to correctly extract the true value of the optical property at $v_{11}$ and $v_{33}$ by compensating the respective values of the photoacoustic signal $s_{11}$ and $s_{33}$ according to the corresponding fluence, encoded in the contribution specifiers $f_{11}$ and $f_{33}$.

Further factors related to the characteristics of the photoacoustic setup which may lead to differences between the domains $v_{11}$ and $v_{33}$ in the amount of sampling light absorbed therein, like for example due to the particular way sampling light propagates through the tissue 10 or to the physical properties of the sampling light itself, can also be accounted for by the corresponding contribution specifiers $f_{11}$ and $f_{33}$. This way, the value of the optical property estimated at a given domain is not determined by the particular geometry of the photoacoustic setup 30, which causes the photoacoustic signal $s_{11}$ to be greater than the photoacoustic signal $s_{33}$. Instead, the values $s_{11}$ and $s_{33}$ are properly taken into account by relating them to the corresponding contribution specifiers $f_{11}$ and $f_{33}$. In other words, the contribution specifiers allow the machine learning algorithm to compensate the fluence such that the true values of the optical property can be induced from the values of the photoacoustic signal.

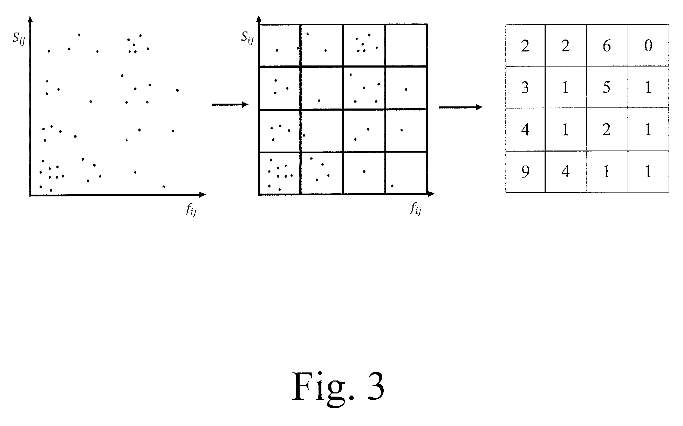

A sequence of training images made up of a large number Q of training images is typically processed by the machine learning algorithm in order to develop and improve the ability to estimate the value of an optical property of a tissue from a photoacoustic image thereof. In that case, a descriptor $F\left(D_V^{(q)}\right)$ is determined from the vector object $D_V^{(q)}$ in the form of a histogram by discretising the two-dimensional feature space of values of the contribution specifiers and values of the photoacoustic signal in bins and counting and storing the number of pairs of values $(f_{ij}, s_{ij})$ comprised in each of the bins. This is illustrated in figure 3. As shown in the leftmost block, the pairs of values $(f_{ij}, s_{ij})$ comprised in the vector object $D_V^{(q)}$ are considered in the feature space of values of the contribution specifiers and the photoacoustic signal. The horizontal axis represents the value of the contribution specifier $f_{ij}$, whereas the vertical axis represents the corresponding value of the photoacoustic signal $s_{ij}$. As shown in the block in the middle, the feature space is discretised in this case in, for instance, 16 bins. The number and size of the bins can be chosen in a convenient way taking into account the particularities of the photoacoustic setup and the tissue 10 and the computational resources available, but must be the same for all domains and all training images.

In the example shown, the number of pairs of values is exaggerated with respect to the examples of previous figures for illustrative proposes. Note however, that the exemplary case presented in Fig. 1 would have 9 pairs of values corresponding to the number of domains comprised in a set of contributing domains therein. Note also, that in real cases, the numbers involved are typically much larger than the ones used in these representative examples.

Once the feature space of values of the contribution specifiers and of the photoacoustic signal has been discretised in bins as shown, the number of pairs of values comprised in each of the bins is counted and stored as a histogram value. As illustrated in the rightmost block, the vector object is thereby transformed in a histogram comprising 16 numerical values corresponding to the number of pairs of values comprised in each of the bins. This histogram is then used as a descriptor to characterise the domain V.

The procedure elucidated above can be repeated for each of the K domains and each of the Q training images, such that a histogram is obtained for each of the K domains and each of the Q training images. Hence a total of K x Q descriptors $F\left(D_V^{(q)}\right)$ in the form of histograms is determined. The set of the K x Q histograms can then be included in a vector object $D = \left\{F\left(D_{V_1}^{(1)}\right), ..., F\left(D_{V_i}^{(q)}\right), ..., F\left(D_{V_K}^{(Q)}\right)\right\}$ that can be analysed by the machine learning algorithm, wherein the component $F\left(D_{V_i}^{(q)}\right)$ corresponds to the histogram descriptor that characterises the domain $V_i$ in the q-th training image.

Hence, the machine learning algorithm characterises each one of the K domains 22 in which the photoacoustic image 20 is partitioned by means of a descriptor $F\left(D_V^{(q)}\right)$ that contains information about the domains $v_{ij}$ comprised in the set of contributing domains $C_V$ defined for the given domain V in the form of a histogram registering the population distribution of the pairs of values $(f_{ij}, s_{ij})$ in the feature space of values of the contribution specifiers and values of the photoacoustic signal.

As pointed out, the way of processing photoacoustic images elucidated above applies to training images of a sequence of training images processed by the machine learning algorithm in the course of a training process. In particular, the photoacoustic image 20 can correspond to a simulated training image. In that case, the characteristics of the tissue 10 and of the photoacoustic setup 30 can be computationally reproduced. Thereby, the value of an optical property, for example the absorption coefficient, of the tissue 10 at each of the domains 22 can be computed, for example by means of an analytic model or of a computer simulation, and compiled in a vector object $A = \left\{ A_{V_1}^{(1)}, ..., A_{V_i}^{(q)}, ..., A_{V_K}^{(Q)} \right\}$ storing the computed - i.e. known - values of the optical property at each of the domains for each of the training images, wherein the component q $A_{V_i}^{(q)}$ corresponds to the value of the optical property at the domain $V_i$ of the q-th training image. Hence the vector object A contains the ground truth values of the optical property used to train the machine learning algorithm according to the principles of supervised learning as described above,. Further, the values of the photoacoustic signal $s_{ij}$ and of the contribution specifiers $f_{ij}$ associated to each of the domains 22 of the photoacoustic image 20 can also be simulated for the tissue 10 and the photoacoustic setup 30, for example by means of Monte Carlo simulations.

Nevertheless, the photoacoustic image 20 can also correspond to a real training image obtained by means of a real photoacoustic measurement. In that case, the matrix object A would comprise entries $A_{V_i}^{(q)}$ corresponding to real values of the absorption coefficient at each of the domains 22 of the photoacoustic image 20. The corresponding values of the photoacoustic signal $s_{ij}$ and of the contribution specifiers $f_{ij}$ could be obtained anyway by means of computer simulations or, additionally or alternatively, by means of an analytic model. For example, in the case of the particularly simple geometry of the photoacoustic setup 30, a point-like light source model can be employed to compute the values of the photoacoustic signal sij and the contribution specifiers $f_{ij}$

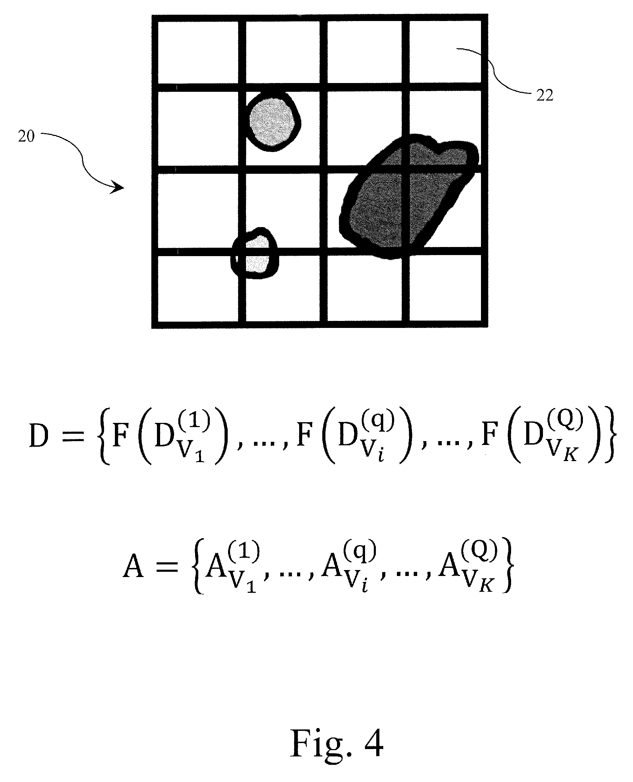

Figure 4 illustrates the situation during a training process, in the course of which Q training images are analysed by the machine learning algorithm. This analysis can be carried out by means of the vector object D comprising the aforementioned K x Q histograms and of the vector object A comprising the corresponding known values of the optical property. The vector objects D and A can be fed into the machine learning algorithm, which is configured for processing them, splitting the data if necessary as required, for example by means of a bootstrap aggregating technique, so as to learn how to infer the optical property of a tissue from a photoacoustic image thereof.

In a way characteristic of this kind of algorithm, the machine learning algorithm is then able to analyse the information contained in the vector objects D and A and to extract from it the underlying causal connections between a photoacoustic image 20 and the corresponding optical properties a tissue 10. The higher the number of training images analysed by the machine learning algorithm, the more accurate and reliable estimations of the value of the optical property of a tissue will be produced by the machine learning algorithm from a photoacoustic image of the tissue. After having been trained on a sufficient amount of training images, the machine learning algorithm develops the ability of inferring the value of the optical property of the tissue from a photoacoustic image thereof by means of the corresponding descriptors. Additional training contributes to further improve this ability. For instance, the inventors have been able to produce reliable results using a set of 100 training images each partitioned in 3000 domains, which amounts to training data comprising 300000 descriptors. Increasing the amount of training data can further contribute to an increased reliability of the results.

The training process described above can be followed by a measuring process during which the previously trained machine learning algorithm profits from the experience accumulated during the training process and is used for the purposes of estimating an optical property. After the completion of the training process, the machine learning algorithm has the ability to infer the value of the optical property at a domain V from the corresponding descriptor $F\left( D_{V_i}^{(q)} \right)$.

Hence in order to estimate the value of the optical property at the at least one domain of the photoacoustic image 20 of a tissue 10, it is only necessary to determine the descriptor or set of descriptors corresponding to said at least one domain of the photoacoustic image 20. For this purpose, values of the photoacoustic signal $s_{ij}$ are measured at each of the domains 22 in which the photoacoustic image 20 is partitioned and incorporated into the corresponding vector objects $D_V^{(measure)}$, wherein the superscript "measure" indicates that the vector object characterises a photoacoustic image of the tissue 10 during the measuring process, i.e. not with the sole purpose of learning, but in order to estimate the value of the optical property corresponding to said photoacoustic image of the tissue 10. If, as is the case in the example under consideration, the characteristics of the photoacoustic setup 30 and of the sampling light reflected by the contribution specifiers $f_{ij}$ have not changed with respect to the training process, contribution specifiers $f_{11},...,-$ determined before the measuring process, for example, during the training process, can be used as part of the descriptor in combination with the newly measured values of the photoacoustic signal $s_{ij}$ in the manner explained above and to determine the corresponding histogram from the resulting pairs of values ($f_{ij}$, $s_{ij}$) that constitutes the descriptor $F\left(D_{V_i}^{(measure)}\right)$, from which the machine learning algorithm can infer the value of the optical property at the domain $V_i$.

Further, for a given tissue like the tissue 10 of Figs. 1 and 2, an optical property can be estimated from a sequence of photoacoustic images like the one 20 in Figs. 1 and 2 obtained using sampling light of different wavelengths. This way, values of, for instance, the absorption coefficient at different wavelengths can be inferred from photoacoustic images obtained using sampling light of the different wavelengths. For example, twenty-six different images of the same tissue obtained at wavelengths ranging between 400 nm and 1600 nm at intervals of 100 nm can be used for this purpose such that corresponding twenty-six values of the absorption coefficient, each at one of the wavelengths ranging between 400 nm and 1600 nm at intervals of 100 nm, can be inferred

Once an optical property, like for example the absorption coefficient, has been determined for a tissue 10, the information can be used to compute other properties of the tissue 10, like for example the oxygenation. When doing so, the machine learning algorithm can take into account the confidence of the inferred optical property at each of the domains 22 of the photoacoustic image 20 of the tissue 10, such that the confidence of the value of the oxygenation obtained at each of the domains 22 in which the photoacoustic image 20 is partitioned is known.

The values of the confidence of the estimation of the optical property at each of the domains 22 can be used to improve the sparsity and the smoothness of the latter by means of spatial regularization, which may comprise means like interpolation and outlier data removal. Further, the values of the confidence of the estimation of the optical property at each of the domains 22 can be used to compute weighted mean values of any quantities related to the optical property or derived from it, wherein the weighting is based on the confidence values.

Although preferred exemplary embodiments are shown and specified in detail in the drawings and the preceding specification, these should be viewed as purely exemplary and not as limiting the invention. It is noted in this regard that only the preferred exemplary embodiments are shown and specified, and all variations and modifications should be protected that presently or in the future lie within the scope of protection of the invention as defined in the claims.

LIST OF REFERENCE SIGNS

10      tissue
12      part of the tissue
20      photoacoustic image
22      plurality of domains
30      photoacoustic setup
32      lipid material
V      domain
$C_V$      set of contributing domains
($v_{11}$, ..,$v_{33}$)      contributing domains
$v_{ij}$      domain or contributing domain
$f_{ij}$      contribution specifier
$s_{ij}$      value of photoacoustic signal

A, D, $D_V^{(q)}$      vector objects

$F\left(D_V^{(q)}\right)$      descriptor

**Claims**

1.    A method for estimating an optical property of a tissue (10) from a photoacoustic image (20) of the tissue (10) or parts thereof (12) using a machine learning algorithm, wherein the photoacoustic image (20) is obtained with a photoacoustic setup (30) and wherein the machine learning algorithm is configured to infer the optical property at least at one domain of the photoacoustic image (20) by means of a descriptor for said at least one domain, wherein:

- at least part of the photoacoustic image (20) is partitioned in a plurality of domains (22) with respect to at least one parameter, wherein the at least one parameter corresponds to a physical property of the tissue (10), and wherein the variation of the at least one parameter within each domain is limited to a pre-determined value, such that each domain corresponds to a limited range of said physical property of the tissue (10); and
- a descriptor is determined for each of said at least one domain, wherein the descriptor for a given domain (V) comprises information related to the photoacoustic image (20) for each contributing domain ($v_{11},...,v_{33}$) of a set of contributing domains ($C_V$), wherein the set of contributing domains ($C_v$) comprises one or more domains other than said given domain (V).

2. The method of claim 1, wherein the descriptor further comprises, for each contributing domain of the set of contributing domains ($C_V$), information related to the location of said given domain (V) and each of said contributing domains ($v_n,...,v_{33}$) with regard to the photoacoustic setup (30).

3. The method of claim 2, wherein the information related to the location of said given domain (V) and each of said contributing domains ($v_{11},...,v_{33}$) with regard to the photoacoustic setup (30) is in the form of a contribution specifier ($f_{ij}$), wherein the contribution specifier ($f_{ij}$) for a contributing domain ($v_{ij}$) defines a relationship between said contributing domain ($v_{ij}$) and said given domain (V), wherein the relationship reflects characteristics of the photoacoustic setup (30).

4. The method of claim 3, wherein said characteristics of the photoacoustic image (20) comprise at least one of the geometry of the photoacoustic setup (30), the properties of the sampling light, and the effects of light propagation through the tissue (10).

5. The method of any of claims 1 to 4, wherein the information related to the photoacoustic image (20) is comprised in a value of a photoacoustic signal ($s_{ij}$), wherein the photoacoustic signal is evaluated at least at some of said plurality of domains (22), and wherein the value of the photoacoustic signal ($s_{ij}$) at a given domain ($v_{ij}$) is a measure of the energy of the sound waves resulting from the photoacoustic effect in that given domain ($v_{ij}$).

6. The method of any of claims 1 to 5, further comprising a training process of the machine learning algorithm, wherein during the training process the machine learning algorithm analyses a sequence of training images and learns how to infer an optical property of a tissue (10) from a photoacoustic image (20) of the tissue (10) using said descriptors, wherein the sequence of training images comprises photoacoustic images of a tissue (10) and values of the optical property of said tissue (10);
wherein the sequence of training images preferably comprises simulated photoacoustic images, wherein a simulated photoacoustic image is a photoacoustic image (20) obtained at least in part by computer simulation; and/or
wherein the sequence of training images preferably comprises real photoacoustic images, wherein a real photoacoustic image is a photoacoustic image (20) obtained by means of a photoacoustic imaging process; and/or
wherein transfer learning means are used to combine training images comprising simulated photoacoustic images and training images comprising real photoacoustic images.

7. The method of any of claim 6, further comprising a measuring process after the training process, wherein during or after the measuring process, the machine learning algorithm infers the optical property at least at one domain of the photoacoustic image (20) from the descriptor determined for said at least one domain;
wherein the information related to the location of said given domain (V) and each of said contributing domains ($v_{11},...,v_{33}$) with regard to the photoacoustic setup (30) for each contributing domain ($v_{11},...,v_{33}$) of the set of contributing domains ($C_V$) or the contribution specifier ($f_{11},...,f_{33}$) for each contributing domain ($v_{11},...,v_{33}$) of the set of contributing domains ($C_V$) is preferably determined before the measuring process; and/or
wherein the information related to the photoacoustic image (20) for each contributing domain ($v_{11},...,v_{33}$) of a set of contributing domains ($C_v$) or the value of the photoacoustic signal ($s_{11},...,s_{33}$) for each contributing domain ($v_{11},...,v_{33}$) of a set of contributing domains ($C_V$) is obtained during the measuring process.

8. The method of claims 3, 5, and any of 4, 6, and 7, wherein the descriptor $F\left(D_V^{(q)}\right)$ for a given domain (V) is determined as a histogram registering values of the contribution specifier ($f_{ij}$) and of the photoacoustic signal ($s_{ij}$), wherein, for one or more training images of the sequence of training images, the machine learning algorithm preferably analyses a vector object (D) comprising histograms for each of said at least one domain and a vector object (A) comprising corresponding values of the optical property at each of said at least one domain.

9. The method of any of the preceding claims, wherein the machine learning algorithm is further configured for estimating a confidence of the optical property at each of the at least one domains of the photoacoustic image (20).

**10.** The method of any of claims 3 to 9, wherein the contribution specifier ($f_{ij}$) for a contributing domain ($v_{ij}$) of a given domain (V) is related to the degree to which the value of the photoacustic signal ($s_{ij}$) and/or of the optical property at said contributing domain ($v_{ij}$) is related to the value of the photoacoustic signal ($s_{ij}$) and/or of the optical property at said given domain (V) for a given photoacoustic setup (30); and/or

wherein the contribution specifier ($f_{ij}$) for said given domain (V) and for said contributing domain ($v_{ij}$) is related to the likelihood that sampling light used for obtaining the photoacoustic image (20) with a given photoacoustic setup (30) reaching said given domain (V) has previously reached said contributing domain ($v_{ij}$); and/or wherein at least one contribution specifier ($f_{ij}$) is computed by means of an analytic model for a given photoacoustic setup (30); and/or wherein at least one contribution specifier ($f_{ij}$) is computed by means of computer simulation.

**11.** The method of any of the preceding claims, wherein the photoacoustic image (20) is partitioned with respect to three parameters, wherein said three parameters correspond to three spatial dimensions of the tissue (10), wherein in particular, the domains correspond to voxels; and/or

wherein the at least one parameter comprises a parameter corresponding to any of a spatial dimension, a time measure, a frequency, and a temperature; and/or

wherein the optical property is estimated from a sequence of photoacoustic images of the tissue obtained using sampling light of different wavelengths; and/or

wherein the sampling light used for obtaining the photoacoustic image comprises light of wavelengths between 400 nm and 1600 nm; and/or

wherein the optical property corresponds to or is at least related to any of the fluence, the absorption coefficient and the optical absorption; and/or

wherein the photoacoustic image (20) is obtained by means of any of photoacoustic image tomography, photoacoustic image microscopy and photoacoustic elastography; and/or

wherein the machine learning algorithm comprises any of a deep learning algorithm, a random forest algorithm, a support vector machine algorithm and a convolutional neural network algorithm.

**12.** A data-processing apparatus comprising means for carrying out the method of any of claims 1 to 11.

**13.** A computer program product including executable code which when executed on a computer carries out the method of any of claims 1 to 11.

**14.** A computer program product including executable code which when executed on a computer estimates an optical property of a tissue (10) from a photoacoustic image (20) of the tissue (10) or a part thereof (12) using a machine learning algorithm, wherein the photoacoustic image (20) is obtained with a photoacoustic setup (30) and wherein the machine learning algorithm is configured to infer the optical property at least at one domain of the photoacoustic image (20) by means of a descriptor for said at least one domain, wherein

- at least part of the photoacoustic image (20) is partitioned in a plurality of domains (22) with respect to at least one parameter, wherein the at least one parameter corresponds to a physical property of the tissue (10), and wherein the variation of the at least one parameter within each domain is limited to a predetermined value, such that each domain corresponds to a limited range of said physical property of the tissue (10);
- the descriptor for a given domain (V) comprises:

i. information related to the photoacoustic image for each contributing domain ($v_{11},...,v_{33}$) of a set of contributing domains ($C_V$), which information is obtained from the photoacoustic image (20), and
ii. information related to the location of said given domain (V) and each of said contributing domains ($v_{11},...,v_{33}$) with regard to the photoacoustic setup (30), which information is comprised in the executable code;

wherein

• the set of contributing domains ($C_v$) comprises one or more domains other than said given domain (V); and
• the information related to the location of said given domain (V) and each of said contributing domains ($v_{11},...,v_{33}$) with regard to the photoacoustic setup (30) preferably is in the form of a contribution specifier ($f_{ij}$), wherein the contribution specifier ($f_{ij}$) for a contributing domain ($v_{ij}$) defines a relationship between said contributing domain ($v_{ij}$) and said given domain (V), wherein the relationship reflects characteristics of the photoacoustic setup (30).

**15.** A computer-readable storage medium comprising the computer program of any of claims 13 to 14.

$$D_V^{(q)} = \begin{cases} f_{11} & \dots & f_{33} \\ s_{11} & \dots & s_{33} \end{cases}$$

Fig. 1

Fig. 2

# Fig. 3

$$D = \left\{ F\left(D_{V_1}^{(1)}\right), \dots, F\left(D_{V_i}^{(q)}\right), \dots, F\left(D_{V_K}^{(Q)}\right) \right\}$$

$$A = \left\{ A_{V_1}^{(1)}, \dots, A_{V_i}^{(q)}, \dots, A_{V_K}^{(Q)} \right\}$$

Fig. 4

Europäisches
Patentamt

European
Patent Office

Office européen
des brevets

# EUROPEAN SEARCH REPORT

Application Number

EP 16 17 7204

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MALONE EMMA ET AL: "Multispectral reconstruction methods for quantitative photoacoustic tomography", PROGRESS IN BIOMEDICAL OPTICS AND IMAGING, SPIE - INTERNATIONAL SOCIETY FOR OPTICAL ENGINEERING, BELLINGHAM, WA, US, vol. 9708, 15 March 2016 (2016-03-15), pages 970827-970827, XP060064626, ISSN: 1605-7422, DOI: 10.1117/12.2212440 ISBN: 978-1-5106-0027-0 * sections 1 and 2; figure 3 * | 1-15 | INV. G06K9/00 |
| X | SILVIA DELSANTO ET AL: "Characterization of a Completely User-Independent Algorithm for Carotid Artery Segmentation in 2-D Ultrasound Images", IEEE TRANSACTIONS ON INSTRUMENTATION AND MEASUREMENT, IEEE SERVICE CENTER, PISCATAWAY, NJ, US, vol. 56, no. 4, 1 August 2007 (2007-08-01), pages 1265-1274, XP011187927, ISSN: 0018-9456, DOI: 10.1109/TIM.2007.900433 * Section II; figure 2 * | 1-15 | |
| | | | TECHNICAL FIELDS SEARCHED (IPC) |
| | | | G06K |
| X | COX B T ET AL: "TWO-DIMENSIONAL QUANTITATIVE PHOTOACOUSTIC IMAGE RECONSTRUCTION OF ABSORPTION DISTRIBUTIONS IN SCATTERING MEDIA BY USE OF A SIMPLE ITERATIVE METHOD", APPLIED OPTICS, OPTICAL SOCIETY OF AMERICA, WASHINGTON, DC; US, vol. 45, no. 8, 10 March 2006 (2006-03-10), pages 1866-1875, XP001241181, ISSN: 0003-6935, DOI: 10.1364/AO.45.001866 * sections 2 and 3; figure 1 * | 1-15 | |

The present search report has been drawn up for all claims

| Place of search | Date of completion of the search | Examiner |
|---|---|---|
| The Hague | 16 December 2016 | Grigorescu, Simona |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................
& : member of the same patent family, corresponding document

EPO FORM 1503 03.82 (P04C01)